# EUROPÄISCHE PATENTANMELDUNG

(11) **EP 3 178 388 A1**
(43) Veröffentlichungstag der Anmeldung: **14.06.2017**
(21) Anmeldenummer: 15199185.8
(22) Anmeldetag: 10.12.2015
(51) Int. Cl.: A61B 5/022

(54) **BLUTDRUCKMESSVORRICHTUNG**

(71) Anmelder: Engels, Bernd, 72760 Reutlingen (DE)
(72) Erfinder: Engels, Bernd, 72760 Reutlingen (DE)
(74) Vertreter: Otten, Roth, Dobler & Partner mbB Patentanwälte

(57) **Zusammenfassung**

Vorgeschlagen wird eine Blutdruckmessvorrichtung (1) zur Messung des Blutdrucks, insbesondere des arteriellen Drucks einer zu untersuchenden Person mit einer Manschette (2), die dazu ausgebildet ist, entlang ihrer Längsrichtung um ein Körperglied, insbesondere den Arm oder das Handgelenk der Person, gelegt und dort arretiert zu werden, sowie einem aufblasbaren Behälter (5) zum Abdrücken des Blutflusses, welche dadurch gebildet ist, dass die Manschette (2) wenigstens zweiteilig (3, 4) ausgebildet ist, indem sie wenigstens ein Manschettenteil (3) und ein Verlängerungsstück (4) zur Verlängerung der Größe in Längsrichtung aufweist, welche durch wenigstens zwei Verbindungsvorrichtungen (9A, 9B, 10A, 10B) jeweils lösbar und wiederverschließbar miteinander in Längsrichtung verbindbar sind.

## Beschreibung

Die Erfindung betrifft eine Blutdruckmessvorrichtung zur Messung des Blutdrucks nach dem Oberbegriff des Anspruchs 1.

Aus dem Stand der Technik sind zahlreiche Blutdruckmessvorrichtungen, insbesondere in Form von Manschetten bekannt. Um aufwändige Reinigungen derartiger Manschetten vermeiden zu können, sind darunter auch Einmal-Manschetten bzw. Wegwerf-Manschetten verfügbar, wie sie auch beispielsweise in der WO 2010/135216 A1 beschrieben sind.

Aufgabe der Erfindung ist es, eine Blutdruckmessvorrichtung bereitstellen zu können, die eine erhöhte Flexibilität beim täglichen Gebrauch ermöglicht.

Die Aufgabe wird, ausgehend von einer Blutdruckmessvorrichtung der eingangs genannten Art, durch die kennzeichnenden Merkmale des Anspruchs 1 gelöst.

Durch die in den abhängigen Ansprüchen genannten Maßnahmen sind vorteilhafte Ausführungen und Weiterbildungen der Erfindung möglich.

Die erfindungsgemäße Blutdruckmessvorrichtung ist zur Messung des Blutdrucks, insbesondere des arteriellen Drucks einer zu untersuchenden Person ausgebildet und umfasst eine Manschette, welche entlang ihrer Längsrichtung um ein Körperglied, in der Regel um den Arm (am Oberarm im Bereich des Ellenbogengelenks) oder gegebenenfalls das Handgelenk der Person gelegt werden kann. Dort wird die Manschette zunächst befestigt bzw. arretiert, sodass die Manschette dicht am Körperglied anliegt. Bei der Messung wird anschließend ein aufblasbarer Behälter zum Abdrücken des Blutflusses aufgeblasen, damit der Raum, der von der Manschette umschlossen und vom Körperglied durchsetzt wird, eingeschnürt wird, sodass ein Druck auf das entsprechende Körperglied ausgeübt wird. Ist der Druck groß genug, so bedeutet dies ein vollständiges Abdrücken des Blutflusses.

Die Manschette der erfindungsgemäßen Blutdruckmessvorrichtung sieht nunmehr wenigstens zwei Teile vor, von denen eines oder wenigstens eines als Manschettenteil und wenigstens eines als Verlängerungsstück zur Verlängerung der Größe in der Längsrichtung bezeichnet wird. Diese Verlängerungsmöglichkeit dient dazu, dass die Blutdruckmessvorrichtung flexibel für verschiedene Durchmesser des entsprechenden Körpergliedes bzw. Arms verwendet werden kann. Manschettenteil und Verlängerungsstück sind durch wenigstens zwei Verbindungsvorrichtungen jeweils lösbar und wiederverschließbar miteinander in Längsrichtung verbindbar. Auf diese Weise kann die Manschette schnell und auch reversibel auf verschiedene Größen eingestellt werden. Manschettenteil und Verlängerungsvorrichtung können meistens an zwei Stellen jeweils miteinander verbunden werden, um einen geschlossenen Ring zu bilden; an jeder der wenigstens zwei Stellen ist daher eine Verbindungsvorrichtung vorgesehen.

Durch das zusätzliche, separate Verlängerungsstück können erfindungsgemäß mehrere Vorteile erreicht werden:
- Bei einer lediglich einteiligen Manschette ohne Verlängerungsstück ist die Möglichkeit einer Größenverstellung stark eingeschränkt. Bei der erfindungsgemäßen Blutdruckmessvorrichtung ist es nicht mehr notwendig, dass dem behandelnden Arzt eine Vielzahl unterschiedlicher Manschetten zur Verfügung steht, damit bei unterschiedlichen Patienten mit unterschiedlich dicken Armen jeweils die passende Manschette vorhanden ist. Denn auch dann, wenn eine Manschette z.B. über einen breitflächigen Klettverschluss zum Verschließen verfügt, kann die Einstellung lediglich in einem eng umgrenzten Rahmen erfolgen. Diese Verstellmöglichkeit wird aber erweitert, wenn zusätzlich eine Verlängerungsstück zur Verfügung steht.
- Die Manschette der erfindungsgemäßen Blutdruckmessvorrichtung kann entsprechend nicht nur verkleinert, sondern auch vergrößert werden.
- Im Unterschied zu aus dem Stand der Technik bekannten, herkömmlichen Manschetten sind, kann gemäß der Erfindung vermieden werden, dass bei Manschetten, die eigentlich zu groß für den Arm des Patienten sind, ein überschüssiger Teil der Manschette überlappt und gegebenenfalls herunterhängt, was beim Blutdruckmessen eher störend wirkt. Bei der erfindungsgemäßen Blutdruckmessvorrichtung kann stattdessen das Verlängerungsstück abgenommen und bei Bedarf wieder an das Manschettenteil angefügt werden.

Grundsätzlich kann die erfindungsgemäße Blutdruckmessvorrichtung lediglich als einteilige Manschette unter Verwendung des Manschettenteils benutzt werden. Je nach Ausführungsform der Erfindung kann zudem das Manschettenteil selbst etwa durch einen breitflächigen Klettverschluss in seiner Größe variabel eingestellt werden. Dann aber, wenn das vorhandene Manschettenteil alleine zur Blutdruckmessung nicht ausreicht, weil der Arm zu dick ist, kann zusätzlich das Verlängerungsstück eingesetzt werden.

Zu diesem Zweck wird das Verlängerungsstück mit dem Manschettenteil in Längsrichtung verbunden. Damit die Manschette den Arm entlang seines Umfangs vollständig umschließen kann, ist sie in einer Richtung, welche hier als Längsrichtung bezeichnet ist, meistens länger ausgebildet als in der Breite. Die Längsrichtung bezeichnet in diesem Zusammenhang also die Längenausdehnung der Manschette, die später um den Umfang des Körperglieds bzw. des Arms reicht.

Insbesondere ist es durch die erfindungsgemäße Maßnahme auch möglich, Blutdruckmessvorrichtungen bzw. Manschetten sogenannter Zwischengrößen bereitzustellen. Da die einzelnen Manschettengrößen, welche z.B. mit dem Buchstaben S (small), M (middle), L (large) usw. bezeichnet und entsprechend eingeteilt werden, festen Längen zugeordnet sind, sind Zwischengrößen oftmals nicht berücksichtigt und können lediglich insoweit abgedeckt werden, wie es die Variabilität der Verbindungsvorrichtung, die oft in Form eines Klettverschlusses vorliegt, dies ermöglicht. Zuweilen werden auch zusätzlich herkömmliche Manschetten für Zwischengrößen angeboten, die beispielsweise mit einem Pluszeichen gekennzeichnet werden, also etwa "M+" für eine Manschette, die etwas größer ist als eine herkömmliche Manschette der Größe "M", nicht aber so groß wie eine Manschette der Größe "L". Aber auch diese herkömmlichen Manschetten besitzen wiederum den Nachteil, dass auch für diese Zwischengrößen somit weitere vollständige Manschetten bzw. Blutdruckmessvorrichtungen gekauft und bereitgestellt werden müssen.

Erfindungsgemäß können darüber hinaus auch mehrere verschiedene Verlängerungsstücke bereitgestellt werden, um eine Erweiterung der Größenskala zu erreichen. An das Manschettenteil werden sodann nicht nur ein, sondern mehrere Verlängerungsstück angebracht.

Das Verlängerungsstück selbst kann sehr einfach gefertigt sein, da es insbesondere nicht notwendig ist, dass das Verlängerungsstück z.B. eine Tasche zur Aufnahme des aufblasbaren Behälters oder eine Schlauchdurchführung oder dergleichen enthält. Dadurch kann das Verlängerungsstück grundsätzlich wesentlich kostengünstiger gefertigt werden als ein Manschettenteil, welches mit entsprechenden Taschen, Durchführungen usw. versehen ist.

Erfindungsgemäß sind wenigstens zwei Verbindungsrichtungen vorgesehen. Das Verlängerungsstück wird zunächst über eine der Verbindungsvorrichtungen an das Manschettenteil gekoppelt. Zum Schließen der Manschette, sodass diese also einen geschlossenen Ring bildet, ist daher eine zweite Verbindungsvorrichtung vorgesehen, die ebenfalls eine Verbindung zwischen dem Manschettenteil und der Verlängerungsvorrichtung herstellen kann. Eine Verbindungsvorrichtung kann entsprechend bei einer Ausführungsform der Erfindung jeweils einen miteinander zusammenwirkendes erstes und zweites Verbindungsteil aufweisen, von denen ein erstes Verbindungsteil am Manschettenteil und ein zweites Verbindungsteil am Verlängerungsstück angeordnet ist, sodass beide miteinander durch deren Zusammenwirkung verbindbar sind.

Bei einem Klettverschluss z.B. könnte eines der beiden Verbindungsteile ein Haken- oder Pilzkopfband sein, während das andere, damit zusammenwirkende Verbindungsteil ein Flauschband, Filzband, Velours-Band oder dergleichen darstellt, sodass die Haken bzw. Pilzköpfe dort einhaken können. Wenn wiederum bei einer Ausführungsform der Erfindung das Manschettenteil und das Verbindungsstück jeweils ein erstes und ein zweites Verbindungsteil aufweisen, so können beide zu einem geschlossenen Ring miteinander verbunden werden. Gleichzeitig bietet dies den Vorteil, dass dann, wenn das Verlängerungsstück nicht benötigt wird, das Manschettenteil immer noch ein erstes und zweites Verbindungsteil aufweist und somit alleine als geschlossener Ring verbindbar ist. Folglich liegt eine besonders flexibel einsetzbare Blutdruckmessvorrichtung vor.

Zur schnellen Verbindung zwischen Manschettenteil und Verlängerungsstück kann dazu das Verlängerungsstück auf einer Seite, welche dem Manschettenteil zuzuwenden ist, ein als zweites Verbindungsteil ausgebildetes Verbindungsteil aufweisen, während auf der gegenüberliegenden Seite ein als erstes Verbindungsteil ausgebildetes Verbindungsteil vorgesehen ist. Insbesondere können beim Manschettenteil und beim Verlängerungsstück die jeweiligen Verbindungsteile auf gegenüberliegenden Flächen angebracht werden, sodass problemlos ein einfaches und schnelles Zusammenfügen zu einem ringartigen Gebilde möglich ist.

Besonders kostengünstige Möglichkeiten, um eine entsprechende, auch schnell und leicht lösbare Verbindung herzustellen, kann die Verbindungsvorrichtung als Klettverschluss bzw. als Druckknopfverschluss mit wenigstens einem Druckknopf ausgebildet sein. Ein Klettverschluss ist vor allem deshalb vorteilhaft, weil er vergleichsweise weich und flexibel ist und zusätzlich keine wesentliche Verdickung der Manschette mit sich bringt, insbesondere keine singulären, festen Punkte ausbildet, sodass beim Zusammenschnüren der Manschette der Klettverschluss in der Regel nicht störend wirkt und auch keine Druckstellen auf dem Arm entstehen. Ein Klettverschluss kann ohne großen Aufwand einfach und schnell verbunden und wieder gelöst werden. Darüber hinaus sind derartige Verbindungsmöglichkeiten in der Regel sehr kostengünstig.

Bei einer Ausführungsvariante können sowohl Manschettenteil als auch Verlängerungsstück grundsätzlich eine Hülle zur Aufnahme des aufblasbaren Behälters und/oder einer Druckmesseinrichtung aufweisen, welche darin einbringbar bzw. entnehmbar ist. Um das Verlängerungsstück jedoch besonders kostengünstig fertigen zu können und das Manschettenteil gewissermaßen universell für verschiedene Größen einsetzen zu können, ist es vorteilhaft, dass lediglich das Manschettenteil eine Hülle zur Aufnahme des aufblasbaren Behälters bzw. der Druckmessvorrichtung aufweist. Durch diese Maßnahme können insbesondere Kosten eingespart werden. Der aufblasbare Behälter kann grundsätzlich aber auch in das Manschettenteil integriert sein, wenn das Manschettenteil z.B. aus einem luftdichten und druckfesten Material besteht oder in das Manschettenteil zur permanenten Anwendung ein entsprechender luftdichter und druckfester Behälter integriert bzw. eingenäht ist. Ein entnehmbarer Behälter ist in der Regel dann vorteilhaft, wenn das Manschettenteil im Sinne der Hülle der Manschette als Wegwerfartikel ausgebildet ist.

Der Vorteil eines zum Dauereinsatz geeigneten Blutdruckmessgeräts bzw. einer entsprechenden Manschette ist es wiederum, dass eine solche Manschette wiederum umweltfreundlicher ist als eine Wegwerfmanschette. Das Manschettenteil und das Verlängerungsstück können darüber hinaus auch beide aus unterschiedlichen Materialien gefertigt sein, wobei z.B. das Manschettenteil auch zum Dauereinsatz ausgebildet sein kann, während das Verlängerungsstück selbst als Wegwerfartikel bzw. Einmalartikel gefertigt ist. Mit diesen verschiedenen Ausführungsvarianten kann einerseits Umweltaspekten Rechnung getragen werden: Die Menge an Wegwerfartikeln, die grundsätzlich die Umwelt stärker belasten können als zum Dauereinsatz geeignete Artikel, kann reduziert werden; andererseits wiederum kann auch die Zahl solcher Artikel verringert werden, die einzeln und besonders aufwändig sterilisiert werden müssen. Grundsätzlich können aber auch Manschettenteil und Verlängerungsstück beide z.B. als Wegewerf- oder Einmalartikel ausgebildet sein. In diesem Fall ist es vorteilhaft, dass als Material des Wegwerfartikels ein recycelbares Material verwendet wird, um wiederum die Umweltbelastung möglichst gering zu halten.

Zur Feineinstellung kann bei einer Weiterbildung der Erfindung jeweils das erste und/oder das zweite Verbindungsteil jeweils am Manschettenteil bzw. am Verlängerungsstück mehrfach hintereinander in Längsrichtung angeordnet sein, um die Längenvariabilität zu erhöhen. Wenn es sich bei den Verbindungsteilen z.B. um solche mit einem Klettverschluss handelt, können z.B. die jeweiligen Klettverschlussflächen eine entsprechende Länge aufweisen, damit in diesem Bereich der Manschettenring praktisch an beliebiger Stelle stufenlos geschlossen werden kann. Im Falle eines Klettverschlusses können diese Flächenstücke daher auch durchgehend bzw. kontinuierlich ausgebildet sein, weil die entsprechenden Haken überall am Flauschband eingreifen und sich verhaken können. Denkbar ist es aber auch, dass einzelne Streifen hintereinander angeordnet werden, also keine durchgängige Fläche vorliegt. Ist statt eines Klettverschlusses z.B. ein Druckknopfverschluss vorgesehen, ist die Befestigung ohnehin nur an einzelnen, diskreten Stellen möglich.

Die erfindungsgemäße Vorrichtung bzw. Ausführungsbeispiele der Erfindung können darüber hinaus auch die Möglichkeit bieten, dass mehrere Verlängerungsstücke hintereinander angeordnet werden, um somit die Länge der Manschette insgesamt noch einmal vergrößern zu können. Insbesondere dann, wenn an jedem Verlängerungsstück sowohl ein erstes als auch ein zweites Verlängerungsstück vorgesehen ist, können diese jeweils abwechselnd angeordnet werden, sodass an ein Verlängerungsstück auch ein weiteres angeheftet wird, dessen Ende wiederum mit dem Manschettenteil verbindbar ist. Durch diese Maßnahme kann bezüglich der Gesamtlänge eine beliebige Flexibilität erreicht werden.

### Ausführungsbeispiel

Ein Ausführungsbeispiel der Erfindung ist in den Zeichnungen dargestellt und wird nachstehend unter Angabe weiterer Einzelheiten und Vorteile näher erläutert. Im Einzelnen zeigen:

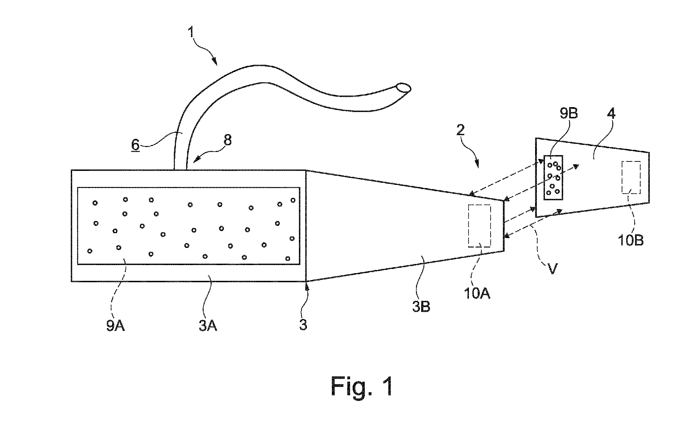

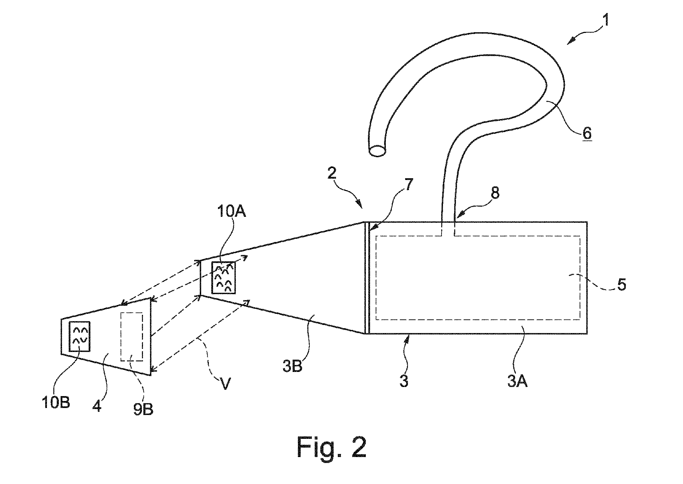

- Figuren 1 und 2:: eine schematische Darstellung einer Blutdruckmessvorrichtung gemäß der Erfindung, jeweils mit einer Ansicht der Vorder- und der Rückseite.

Figur 1 zeigt eine Blutdruckmessvorrichtung 1 gemäß der Erfindung in Form einer Manschette 2, welche aus zwei Teilen besteht, nämlich einem Manschettenteil 3 und einem Verlängerungsstück 4. Das Manschettenteil 3 umfasst einen Bereich 3A, in dem ein aufblasbarer Behälter 5 zum Abdrücken des Blutflusses untergebracht ist sowie eine Lasche 3B, die lediglich zur Verbindung dient. Die Bereiche 3A und 3B sind fest und nicht lösbar miteinander verbunden. Am oberen Rand des Bereichs 3A ist der Textilstoff, aus dem fast die gesamte Manschette 2 gefertigt ist, teilweise unterbrochen, sodass dort eine Durchführung entsteht. Diese Durchführung dient dazu, dass der Schlauch 6 hindurchgeführt werden kann und mit einem weiteren Gerät, das in der Regel ein Druckmessgerät sowie eine Vorrichtung zum Aufblasen und zum Ablassen von Luft umfasst, verbindbar ist. Bei Handmessgeräten handelt es sich dabei in der Regel um einen Handblasebalg mit entsprechender Ventilvorrichtung und ein daran gekoppeltes Manometer.

Die gesamte Manschette 2, welche das Manschettenteil 3 und das Verlängerungsstück 4 umfasst, ist als Einmalartikel bzw. Wegwerfartikel ausgebildet. Der aufblasbare Behälter 5 wiederum kann mehrfach verwendet werden. Der aufblasbare Behälter 5 samt Schlauch 6 kann dazu über die Öffnungslasche 7 in das Manschettenteil 3 gesteckt werden, während der Schlauch 6 über die Öffnung 8 am oberen Rand nach außen geführt werden kann.

Das Manschettenteil 3 weist ein erstes Verbindungsteil 9A und ein zweites Verbindungsteil 10A auf, während das Verlängerungsstück 4 ebenfalls ein erstes Verbindungsteil 9B und ein zweites Verbindungsteil 10B besitzt. Die Verbindungsteile 9A und 9B (erste Verbindungsteile) sind als Flauschbänder ausgebildet, während die Verbindungsteile 10A und 10B (zweite Verbindungsteile) als Hakenbänder ausgebildet sind. Erstes und zweites Verbindungsteil ergeben somit einen Klettverschluss. Um eine möglichst variable Einstellung der Größe zu ermöglichen, ist das Flauschband 9A in Längsrichtung sehr langgestreckt ausgebildet, sodass in diesem Bereich eine stufenlose Einstellung durch Anheften des Hakenbandes 10B erfolgen kann. Das Manschettenteil 3 und das Verlängerungsstück 4 werden über die beiden Verbindungsteile 10A und 9B miteinander verbunden. Diese Verbindung ist in den Figuren 1 und 2 durch gestrichelte Doppelpfeile gekennzeichnet. Die Haken der zweiten Verbindungsteile 10A und 10B können in die Flauschbänder 9A und 9B einhaken, sodass sich eine einfach herzustellende, feste, aber auch leicht lösbare Verbindung ergibt. Diese Verbindung ist fest in Längsrichtung der Manschette 2, also parallel zur Fläche der Verbindungsteilbänder, während bei einer Kraftwirkung senkrecht zur Fläche die Bänder wieder getrennt werden können. Somit umfasst die Manschette 3 ein erstes und zweites Verbindungsteil 9A und 10A, während auch das Verlängerungsstück 4 wiederum ein erstes Verbindungsteil 9B und ein zweites Verbindungsteil 10B aufweist. Bei Bedarf könnte an das Verbindungsteil 10B ein weiteres Verlängerungsstück mit einem entsprechenden Flauschband angeheftet werden. In Figur 1 befinden sich die Hakenbänder 10A und 10B auf der Rückseite, sodass diese gestrichelt eingezeichnet sind. Analog ist in Figur 2 das Flauschband 9B gestrichelt eingezeichnet. Das Verbindungsteil 9A befindet sich wiederum hinter dem aufblasbaren Behälter in Figur 2, sodass dieses nicht weiter eingezeichnet ist.

### Bezugszeichenliste:

- 1: Blutdruckmessvorrichtung
- 2: Manschette
- 3: Manschettenteil
- 3A, 3B: Bereiche am Manschettenteil
- 4: Verlängerungsstück
- 5: aufblasbarer Behälter
- 6: Schlauch
- 7: Öffnungslasche
- 8: Durchführung
- 9A, 9B: erstes Verbindungsteil (Flauschband)
- 10A, 10B: zweites Verbindungsteil (Hakenband)
- V: Verbindung

## Patentansprüche

1. Blutdruckmessvorrichtung (1) zur Messung des Blutdrucks, insbesondere des arteriellen Drucks einer zu untersuchenden Person mit einer Manschette (2), die dazu ausgebildet ist, entlang ihrer Längsrichtung um ein Körperglied, insbesondere den Arm oder das Handgelenk der Person, gelegt und dort arretiert zu werden, sowie einem aufblasbaren Behälter (5) zum Abdrücken des Blutflusses, **dadurch gekennzeichnet, dass** die Manschette (2) wenigstens zweiteilig (3, 4) ausgebildet ist, indem sie wenigstens ein Manschettenteil (3) und ein Verlängerungsstück (4) zur Verlängerung der Größe in Längsrichtung aufweist, welche durch wenigstens zwei Verbindungsvorrichtungen (9A, 9B, 10A, 10B) jeweils lösbar und wiederverschließbar miteinander in Längsrichtung verbindbar sind.

2. Blutdruckmessvorrichtung (1) nach einem der vorgenannten Ansprüche, **dadurch gekennzeichnet, dass** die wenigstens zwei Verbindungsvorrichtungen (9A, 9B; 10A, 10B) jeweils ein miteinander zusammenwirkendes erstes (9A, 9B) und zweites (10A, 10B) Verbindungsteil aufweisen, von denen ein erstes Verbindungsteil am Manschettenteil (3) und ein zweites Verbindungsteil am Verlängerungsstück (4) angeordnet ist, sodass beide miteinander durch die Zusammenwirkung verbindbar sind.

3. Blutdruckmessvorrichtung (1) nach einem der vorgenannten Ansprüche, **dadurch gekennzeichnet, dass** das Manschettenteil (3) und das Verbindungsstück (4) jeweils ein erstes (9A, 9B) und zweites Verbindungsteil (10A, 10B) aufweisen, sodass beide zu einem geschlossenen Ring verbindbar sind.

4. Blutdruckmessvorrichtung (1) nach einem der vorgenannten Ansprüche, **dadurch gekennzeichnet, dass** das Manschettenteil (3) allein ohne Verbindungsstück (4) zu einem geschlossenen Ring verbindbar ist, insbesondere über das am Manschettenteil (3) angebrachte erste (9A) und zweite Verbindungsteil (10A).

5. Blutdruckmessvorrichtung (1) nach einem der vorgenannten Ansprüche, **dadurch gekennzeichnet, dass** das Verlängerungsstück (4) auf einer dem Manschettenteil (3) zuzuwendenden Seite ein als zweites Verbindungsteil (10B) ausgebildetes Verbindungsteil und auf der gegenüberliegenden Seite ein als erstes Verbindungsteil (9B) ausgebildetes Verbindungsteil aufweist.

6. Blutdruckmessvorrichtung (1) nach einem der vorgenannten Ansprüche, **dadurch gekennzeichnet, dass** jeweils beim Manschettenteil (3) und beim Verlängerungsstück (4) die jeweiligen Verbindungsteile (9A, 9B; 10A, 10B) auf gegenüberliegenden Flächen angebracht sind.

7. Blutdruckmessvorrichtung (1) nach einem der vorgenannten Ansprüche, **dadurch gekennzeichnet, dass** die Verbindungsvorrichtung (9A, 9B; 10A, 10B) als Klettverschluss und/oder als Druckknopfverschluss mit wenigstens einem Druckknopf ausgebildet ist/sind.

8. Blutdruckmessvorrichtung (1) nach einem der vorgenannten Ansprüche, **dadurch gekennzeichnet, dass** wenigstens einer der Teile (3, 4) der Manschette (2) eine Hülle zur Aufnahme des aufblasbaren Behälters (5) und/oder einer Druckmesseinrichtung aufweist, in welche der Behälter einbringbar und entnehmbar lagerbar ist.

9. Blutdruckmessvorrichtung (1) nach einem der vorgenannten Ansprüche, **dadurch gekennzeichnet, dass** der Behälter (5) in den Manschettenteil (3) integriert ist.

10. Blutdruckmessvorrichtung (1) nach einem der vorgenannten Ansprüche, **dadurch gekennzeichnet, dass** das Manschettenteil (3) und/oder das Verlängerungsstück (4) aus einem recycelbaren Material gefertigt ist, um als Einmal-Artikel vom Rest der Blutdruckmessvorrichtung getrennt und weggeworfen werden zu können.

11. Blutdruckmessvorrichtung (1) nach einem der vorgenannten Ansprüche, **dadurch gekennzeichnet, dass** das erste Verbindungsteil (9A) und/oder das zweite Verbindungsteil (9B) am Manschettenteil (3) und/oder am Verlängerungsstück (4) mehrfach hintereinander in Längsrichtung angeordnet sind, um die Längenvariabilität zu erhöhen.
